# EUROPEAN PATENT APPLICATION

(11) **EP 0 613 719 A1**
(43) Date of publication of application: **07.09.1994**
(21) Application number: 93200635.6
(22) Date of filing: 05.03.1993
(51) Int. Cl.: B01J 31/24, C07C 253/14, C07F 15/04

(54) **Nickel catalyst for the cyanation of aromatic halides**

(71) Applicant: DUPHAR INTERNATIONAL RESEARCH B.V, NL-1380 AC Weesp (NL); Rijksuniversiteit Utrecht, NL-3584 CS Utrecht (NL)
(72) Inventor: van Soolingen, Jacob, P.O. Box 140 NL-1380 AC Weesp (NL); Brandsma, Lambert, P.O. Box 140 NL-1380 AC Weesp (NL); Kruse, Chris G., P.O. Box 140 NL-1380 AC Weesp (NL)
(74) Representative: Swaters, Pieter D.

(57) **Abstract**

The invention relates to a nickel compound to be used as a catalyst for cyanation reactions, said nickel compound having a central nickel atom complexed by a diphosphine ligand. In said diphosphine ligand the P-atoms are interconnected through an aliphatic, cycloaliphatic or aromatic bivalent radical. The invention also relates to the use of said nickel compound and to a method of preparing an aromatic cyanide from an aromatic halide and an alkali metal cyanide in a substantially anhydrous non-dipolar aprotic solvent under the influence of a phosphine-nickel catalyst and in the presence of a salt of a transition metal.

## Description

The invention relates to a nickel compound to be used as a catalyst for the cyanation, in particular of aromatic halides, and to a method of preparing said compound. The invention further relates to a method of preparing an aromatic cyanide by reacting an aromatic halide with an alkali metal cyanide under the influence of a phosphine-nickel catalyst.

It is well-known that certain nickel complexes can catalyse the cyanation of aromatic halides, i.e. the conversion of aromatic halides into the corresponding aromatic cyanides under the influence of an alkali metal cyanide.

Cassar and coworkers (Adv. in Chem. Series 1974, 132, 252-273) have extensively studied the nickel-catalyzed cyanation of aromatic halides. In addition to nickel(0) complexes with triphenylphosphine and tris(o-tolyl)phosphine as ligands, these authors have investigated the use of nickel, complexed with two molecules 1,4-bis(diphenylphosphino)butane. This last compound appeared to be less effective than the former compounds in the cyanation of 1-chloronaphthalene under the conditions applied, viz. in ethanol at 60°C. This result is not an impetus for further research into the use of diphosphines as ligands for nickel(0) complexes. Also the mechanism of the nickel-catalysed cyanation, as suggested by Cassar et al., is not stimulating for any further investigation in this direction. In this mechanism a five coordinated nickel complex is postulated as an intermediate, wherein the diarylphosphine ligands are perpendicularly positioned on the planar structure, formed by nickel with its remaining ligands, and consequently are in the trans position to each other.

More recently, Cassar and coworkers have reported the nickel-catalyzed cyanation of aromatic halides under phase-transfer conditions: J. Organometal. Chem. 1979, 173, 335-339. The presence of a phase-transfer catalyst, viz. an ammonium salt or a crown ether, should make the process more attractive for the industrial production of aromatic cyanides. Nevertheless, in a suitable solvent, viz. benzene, the cyanation occurs only if the NaCN is added slowly to keep the NaCN-concentration in the aqueous phase below approx. 0.1 mol/l. This is, of course, not favourable for the realization of a process on an industrial scale.

Sakakitara et al. (Bull. Chem. Soc. Jpn. 1988, 61, 1985-1990) suggest, that the cyanide ion itself has an inhibitory action on the catalyst activity. In an attempt to avoid this problem, Sakakitara and coworkers have performed cyanation reactions in polar aprotic solvents, such as HMPA and acetonitrile, wherein the cyanide concentration is low. The cyanation experiments were carried out in the presence of 3 mol% of nickel complex, calculated on starting halide, i.c. chlorobenzene and related compounds. The nickel(0) complexes were generated in situ from NiBr₂ in combination with certain phosphine ligands and in the presence of zinc; in all experiments described, such molar ratios were used that the central nickel atom could be complexed by four phosphine moieties. In addition to triphenylphosphine three other phosphine ligands were used in combination with NiBr₂, viz. tri-o-tolylphosphine, 1,2-bis(diphenylphosphino)ethane and 1,1'-bis(diphenyl-phosphino)ferrocene; the results, however, were not always satisfactory.

In the published European patent application no. 0384392, the cyanation of aromatic halides is disclosed, also under the influence of nickel complex catalysts which are formed during the halide-cyanide conversion. The described cyanations, performed in a number of separate reaction steps, are carried out in anhydrous alcoholic or aprotic solvents and in the presence of a reducing metal, viz. powdered zinc. As will be clear from the examples attached to said published patent application, also in this case at least approximately 2 mol% nickel compound is necessary for performing the desired cyanation. This is a great disadvantage for the realization of a process on a technical scale. In fact, nickel compounds are considered as environmentally highly undesirable and the phosphines applied are expensive materials. Therefore the nickel catalyst should be recovered from the reaction mixture as completely as possible. It will be evident that, if the quantity of the nickel catalyst, required for the cyanation, could be reduced substantially, the working-up procedure after the cyanation reaction could be facilitated considerably. As a consequence, the process could be performed on a technical scale with less expenses and with a reduced environmental risk. In addition, it is described that a large excess of phosphine ligand calculated on starting nickel chloride is apparently necessary to prevent desactivation of the in situ formed nickel catalyst, required for the cyanation reaction. Moreover, dependent on the substituents in the starting aromatic halides, the desired cyanation sometimes does not occur at all by using the known nickel complex catalysts.

It is the objective of the invention to provide a nickel catalyst having a significantly decreased sensitivity for desactivation by cyanide ions and hence an improved effectiveness for the cyanation of aromatic halides.

This objective can be achieved according to the present invention by a nickel compound having the general formula 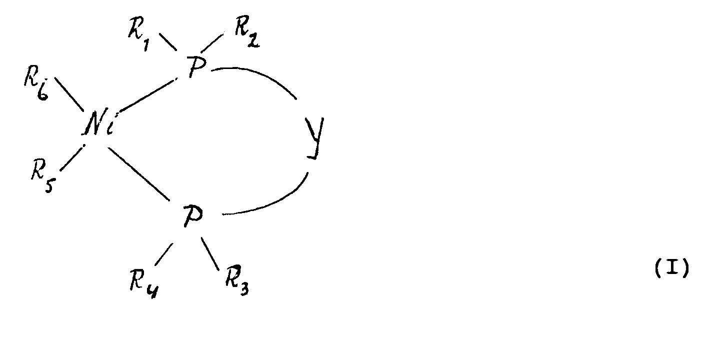
wherein
R₁, R₂, R₃ and R₄ are each independently C₁-C₆ alkyl groups, aryl groups or heteroaryl groups, which (hetero)aryl groups may be substituted with alkyl, halogen, haloalkyl, cyano, amino, N-alkylamino, N,N-dialkylamino, alkoxy or alkylthio, wherein the alkyl groups have 1 to 6 carbon atoms;
Y is an aliphatic, cycloaliphatic or aromatic bivalent radical having 2 to 18 C atoms;
R₅ is a C₁-C₄ alkyl group, an aryl group or a heteroaryl group, which (hetero)aryl group may be substituted with 1-5 substituents, selected from the group consisting of alkyl, alkoxy, alkylthio, haloalkyl, haloalkoxy, halogen, formyl, alkylcarbonyl, optionally substituted phenyl, optionally substituted phenylcarbonyl, optionally substituted phenoxy, optionally substituted phenoxycarbonyl, optionally substituted phenylalkyl, alkoxycarbonyl and cyano, wherein the alkyl groups have 1 to 8 carbon atoms; and
R₆ is a C₁-C₄ alkyl group, an aryl group or a heteroaryl group, which (hetero)aryl group may be substituted with C₁-C₄ alkyl, or a halogen atom selected from Cl, Br and I;
or wherein R₅ and R₆ together constitute a diene molecule having the general formula

R₉ - CH = CH -(CH₂)ₙ - CH = CH - R₁₀

wherein n is 1-4, and
R₉ and R₁₀ are each C₁-C₈ hydrocarbyl, or
R₉ and R₁₀ together constitute a C₁-C₄ alkylene group;
with the provisos,
(1) that R₆ is alkyl if R₅ is alkyl; and
(2) that R₆ is halogen or (hetero)aryl if R₅ is (hetero)aryl.

Surprisingly it has been found, that by using the above new nickel compound of the invention as a catalyst for the cyanation of aromatic halides, of course under suitable reaction conditions, said nickel compound needs only to be added in approx. 0.5 mol% at most, e.g. approx. 0.2 mol%, calculated on starting halide, to reach optimum results as to selective conversion. As will be evident from the accompanying Examples, by using a nickel compound of the invention in such a small quantity, aromatic halides can be converted into the corresponding cyanides in yields of up to 95%. The new nickel compound of the present invention can conveniently be prepared from a nickel halogenide and a diphosphine ligand in stoichiometric quantities (the central nickel atom complexed by one diphosphine ligand), so with an economical ligand consumption. Moreover it has been found, that the new nickel compounds of the invention can successfully catalyse the cyanation of such aromatic halides which cannot be converted under the influence of conventional nickel catalysts.

Suitable substituents for the above phenyl groups in the meanings of R₅ are: alkyl, alkoxy, halogen, haloalkyl and alkylthio, wherein the alkyl groups have 1 to 4 carbon atoms. Suitable examples of aryl groups in the above Formula I compound are phenyl and naphthyl. Suitable examples of heteroaryl groups are: thienyl, pyridyl, furyl, pyrrolyl, N(C₁-C₄)alkylpyrrolyl and thiazolyl. If Y represents an aliphatic bivalent radical, Y may conveniently be ethylene, 1,2-dimethylethylene, trimethylene, tetramethylene or pentamethylene. Aromatic bivalent radicals, which can equally well be used for the symbol Y in the above formula I, include metallocene biradicals, such as the 1,1'-ferrocene biradical, as well as o-phenylene, naphthylene, phenanthrene, 3,4-benzophenanthrene, biphenylene and binaphthylene.
Suitable examples of hydrocarbyl groups for the above substituents R₉ and R₁₀ are alkyl, alkenyl, phenyl and naphthyl.
If R₅ and R₆ together constitute a diene molecule, said diene molecule is attached to the central nickel atom with non-covalent bonds, so-called metal π-complex bonds.

In a preferred embodiment, the nickel compound of the present invention has the general formula 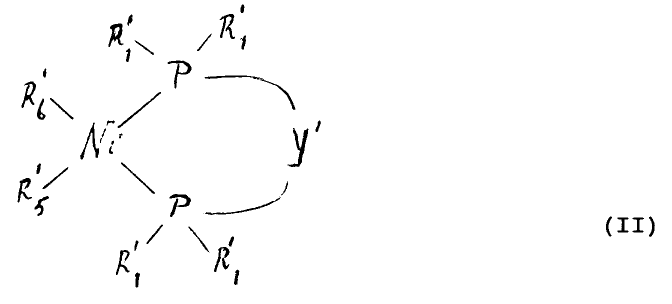
wherein
R₁' is an unsubstituted phenyl group or a phenyl group substituted with C₁-C₄ alkyl, halogen or C₁-C₄ haloalkyl;
Y' is a C₂-C₆ straight or branched alkylene group or a metallocene biradical;
R₅' is a C₁-C₄ alkyl group, an unsubstituted phenyl group or a phenyl group substituted with 1-4 substituents selected from C₁-C₄ alkyl, C₁-C₄ alkoxy, halogen, C₁-C₄ haloalkyl, C₁-C₄ haloalkoxy, formyl, phenyl, phenoxy, C₁-C₄ alkylcarbonyl, cyano, phenylcarbonyl and phenyl(C₁-C₄)alkyl;
or wherein R₅' is a naphthyl group or a heteroaryl group selected from thienyl, pyrrolyl and furyl; and
R₆' is a C₁-C₄ alkyl group or a halogen atom selected from Cl, Br and I; or wherein
R₅' and R₆' together constitute a C₅-C₁₀ cycloalkadiene molecule;
with the provisos,
(1) that R₆' is alkyl if R₅' is alkyl; and
(2) that R₆' is halogen if R₅' is (hetero)aryl.

The new nickel compounds of the present invention can be prepared in a manner known per se for related compounds, preferably as follows.

A nickel compound of the general formula 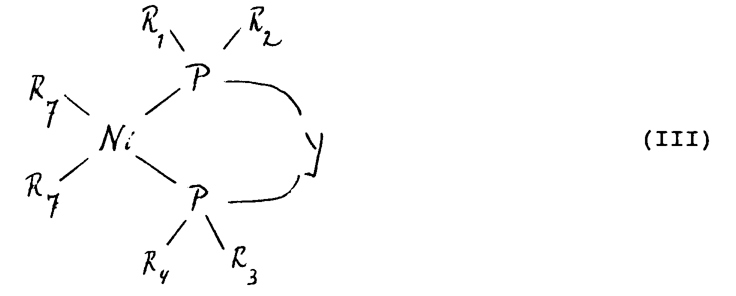
wherein
R₁, R₂, R₃, R₄ and Y have the meanings given above, and
R₇ is a C₁-C₄ alkyl group or a (hetero)aryl group,
can be prepared by reacting a compound of the general formula 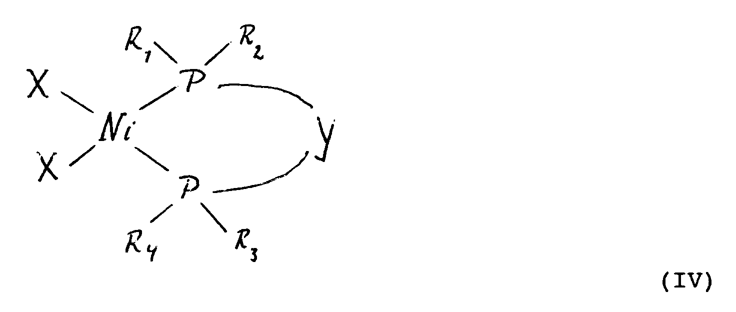
wherein X is a halogen atom selected from Cl, Br and I, with a compound of the general formula

R₇MgX or R₇ZnX (V)

under well-known Grignard conditions.

The starting compounds of the general formula IV can easily be prepared by reacting a nickel halogenide with a diphosphine ligand of the general formula (R₁)₂P-Y-P(R₁)₂ in stoichiometric quantities. This preparation method is conveniently performed as described in the accompanying Examples.

An example of a nickel compound which can be prepared according to the above method is:
(1) diethyl-1,1'-bis(diphenylphosphino)ferrocene-nickel.

Nickel compounds of the above general formula IV are new. Therefore, the present invention also relates to a nickel compound of the general formula IV, wherein the symbols have the above-defined meanings.

A nickel compound of the general formula 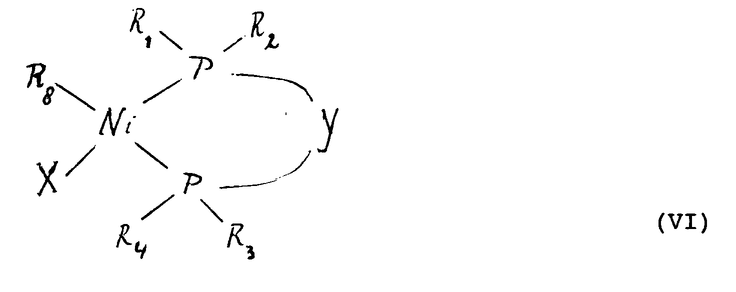
wherein
R₁, R₂, R₃, R₄, X and Y have the meanings given above, and R₈ is an aryl group or a heteroaryl group, which (hetero)aryl group is optionally substituted with one or more substituents, selected from the group consisting of alkyl, alkoxy, alkylthio, haloalkyl, haloalkoxy, halogen, formyl, alkylcarbonyl, optionally substituted phenyl, optionally substituted phenylcarbonyl, optionally substituted phenoxy, optionally substituted phenoxycarbonyl, optionally substituted phenylalkyl, alkoxycarbonyl and cyano, wherein the alkyl groups have 1 to 8 carbon atoms;
can be prepared by reacting a compound of the general formula 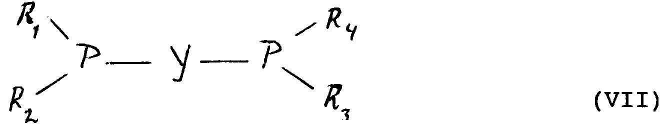
and a compound of the general formula

R₈ - X (VIII)

with NiX₂,
under the influence of a reducing agent.

The reaction is preferably carried out in an organic solvent or solvent mixture, e.g. a mixture of an aromatic hydrocarbon such as toluene with an alcohol such as ethanol. An example of a suitable reducing agent is a metallic reducing agent such as zinc.
Alternatively, the above compound of formula VI can conveniently be prepared by reacting a compound of the general formula III, as defined hereinbefore, with a compound of the general formula VIII, also as defined above, preferably under the same reaction conditions.

Examples of nickel compounds which can be prepared according to the above methods are:
(2) chloro-(4-trifluoromethyl)phenyl-1,1'-bis(diphenylphosphino)-ferrocene-nickel,
(3) chloro-(1-naphthyl)-1,1'-bis(diphenylphosphino)ferrocene-nickel,
(4) chloro-(4-trifluoromethyl)phenyl-1,4-bis(diphenylphosphino)butane-nickel, and
(5) chloro-(2-thienyl)-1,1'-bis(diphenylphosphino)ferrocene-nickel.

A nickel compound of the general formula 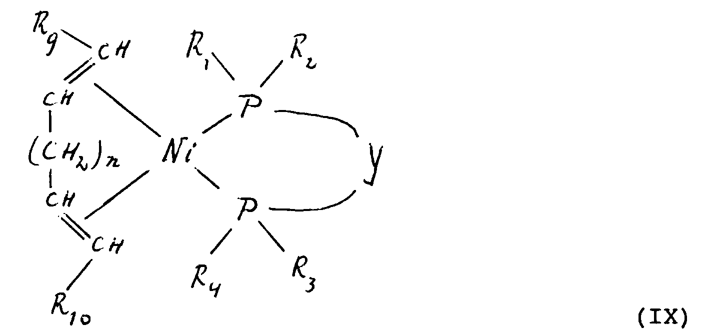
wherein the symbols have the meanings given above,
can be prepared according to two equally attractive methods, viz. either:
(i) by reacting a nickel complex of the general formula 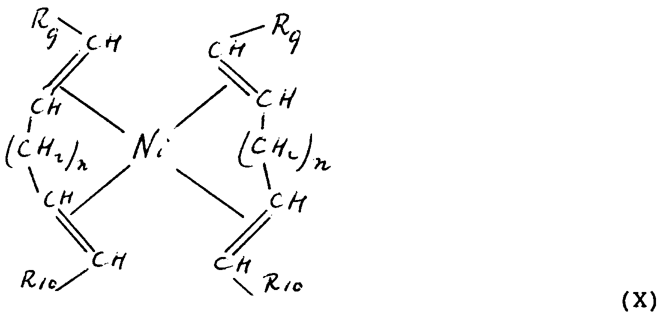 with a compound of the general formula VII, as defined hereinbefore; or
(ii) by reacting a compound of the general formula IV, as defined hereinbefore, with a compound of the general formula

   R₉ - CH = CH - (CH₂)ₙ - CH = CH - R₁₀ (XI)

   under the influence of a reducing agent.

The reaction described sub (i) above is preferably carried out in an aromatic solvent, e.g. toluene, the reaction described sub (ii) above preferably in a polar organic solvent, e.g an alcohol such as ethanol, under the influence of a suitable reducing agent, e.g. a metallic reducing agent such as zinc. An example of a nickel compound which can be prepared according to the last-mentioned method is:
(6) 1,5-cyclooctadiene-1,1'-bis(diphenylphosphino)ferrocene-nickel.

As indicated above, the nickel compounds of the present invention can be used as catalysts for the conversion of aromatic halides of the general formula

Ar - X (XII)

wherein
Ar is an aryl or heteroaryl group, which groups may be substituted with 1-5 substituents, selected from the group consisting of alkyl, alkoxy, alkylthio, haloalkyl, haloalkoxy, halogen, formyl, alkylcarbonyl, optionally substituted phenyl, optionally substituted phenylcarbonyl, optionally substituted phenoxy, optionally substituted phenoxycarbonyl, optionally substituted phenylalkyl, alkoxycarbonyl and cyano, wherein the alkyl groups have 1 to 8 carbon atoms; and
X has the meaning given above;
into an aromatic cyanide of the general formula

Ar - CN (XIII)

under the influence of an alkali metal cyanide.

Suitable substituents for the above phenyl groups are: alkyl, alkoxy, halogen, haloalkyl and alkylthio, wherein the alkyl groups have 1 to 4 carbon atoms.

Suitable examples of aromatic halides which can be used as starting compounds for the above conversion are: chlorotrifluoromethylbenzene, bromo-trifluoromethylbenzene, chlorobenzaldehyde, bromobenzaldehyde, chlorobenzene, bromobenzene, dichlorobenzene, dibromobenzene, trichlorobenzene, dichlorobenzaldehyde, dibromobenzaldehyde, dichloro-trifluoromethylbenzene, dibromo-trifluoromethylbenzene, trichloro-trifluoromethylbenzene, tribromo-trifluoromethylbenzene, chloro-bis(trifluoromethyl)benzene, bromo-bis(trifluoromethyl)benzene, chloro-diformylbenzene, bromo-diformylbenzene, chloroanisole, bromoanisole, chloronaphthalene, chlorophenylbenzene, chlorophenoxybenzene, chloroacetophenone, chlorotoluene, bromotoluene, chlorobenzonitrile, chlorobenzophenone, chloro-fluorobenzene, iodobenzene, bromothiophene, bromofuran, chlorothiophene, bromopyrrole, and the like, including all various position isomers.

As indicated above, the cyanation reaction can be inhibited by contact of the nickel catalyst with dissolved cyanide; apparently under such conditions the catalyst is easily desactivated. Cassar et al. (see above) have shown, that catalyst desactivation can be prevented by keeping the cyanide concentration low, for example, by slowing down the cyanide addition. It will be evident, however, that this cannot be realized favourably in a cyanation process on an industrial scale.

It has now been found surprisingly, that the problem of catalyst desactivation can easily and simply be avoided by performing the cyanation reaction in the presence of a salt of a transition metal, preferably a metal of group 2b of the Periodic Table. Such a simple solution lends itself admirably for a technically attractive realization, resulting in a process wherein the cyanide can be added at once. Consequently, the present invention also relates to a method of preparing an aromatic cyanide of the general formula

Ar - CN (XIII)

wherein Ar has the above meaning,
characterized in that an an aromatic halide of the general formula

Ar - X (XII)

wherein X has also the above meaning,
is reacted with an alkali metal cyanide in a substantially anhydrous non-dipolar aprotic solvent under the influence of a phosphine-nickel catalyst and in the presence of a salt of a transition metal.

An enumeration of suitable starting aromatic halides is presented above.
Suitable phosphine-nickel catalysts for the above reaction are, in addition to the new nickel compounds defined hereinbefore, the following known nickel complexes:
chloro-(4-trifluoromethyl)phenyl-bis(triphenylphosphine)-nickel,
chloro-(4-trifluoromethyl)phenyl-bis[tris(4-trifluorophenyl)phosphine]-nickel,
chloro-(4-trifluoromethyl)phenyl-bis[tris(4-tolyl)phosphine]-nickel,
chloro-(1-naphthyl)-bis(triphenylphosphine)-nickel,
and the like.

Sodium cyanide and potassium cyanide can conveniently be used as a cyanide source. Suitable solvents for the above reaction are tetrahydrofuran, dioxan and methyl tert.-butyl ether; tetrahydrofuran is preferred, because of its solvating properties. Examples of salts of transition metals are: zinc and cadmium salts of mineral acids, such as HCl, H₂SO₄, and the like. The best results are obtained with zinc chloride. It is of advantage, if the reaction mixture in addition contains a reducing agent, preferably powdered zinc, to reach an optimum conversion of the starting halide. Consequently, by using the preferred system - tetrahydrofuran, zinc chloride, powdered zinc, sodium cyanide (completely present in the system), and a suitable nickel catalyst - the desired cyanation can be performed in yields of up to 95%.

The present invention will now be described in greater detail with reference to the following specific Examples.

### Example I

### Preparation of diethyl(1,1'-bis-diphenylphosphinoferrocene)nickel.

The title compound is synthesized under a nitrogen atmosphere; the solvents are dried and deoxygenated before use.
To a suspension of NiCl₂(dppf) (6.84 g; 10 mmoles) in THF (40 ml) is added at -30°C a 1 M solution of ethylmagnesiumbromide in THF (50 ml; 50 mmoles) over 5 min. The mixture is stirred for 0.5 hr at -20°C and allow to warm to room temperature. After filtration, the residue is washed twice with 40 ml toluene. The combined filtrate is concentrated in vacuo to about 20 ml. After adding hexane (100 ml) and cooling to -80°C, the precipitate is filtered and washed twice with 40 ml pentane. The residue is dried in vacuo to yield 8.20 g (94%) of an amorphous solid.
Elem. analysis: C,57.27; H,5.17; Ni,7.2. The analytical results are in conformity with the values calculated for the title compound C₃₈H₃₈FeNiP₂, containing 1.5 mole of ethylmagnesium-bromide: C,56.53; H,5.26; Ni,6.74.
NiCl₂(dppf), necessary for the above reaction, is synthesized under a nitrogen atmosphere. To a solution of 1,1'-bis-diphenylphosphinoferrocene (dppf; 17.20 g; 31 mmoles) in toluene (50 ml) is added a solution of NiCl₂.6H₂O (7.17 g; 30 mmoles) in ethanol (96%; 50 ml) at 50°C over 5 min. The suspension is heated to reflux for 15 minutes and allowed to cool to room temp. After cooling down to 5°C (ice bath), the precipitate is filtered and successively washed with 50 ml toluene, 50 ml ethanol (96%), 50 ml diethylether and 50 ml pentane. The solid is dried in vacuo to yield 18.5 g (90%) NiCl₂(dppf).

### Example II

### Preparation of chloro(1-naphthyl)(1,1'-bis-diphenylphosphinoferrocene)nickel.

The title compound is synthesized under a nitrogen atmosphere. To a solution of 1-chloronaphthalene (16 g; 0.10 mole) and 1,1'-bis-diphenylphosphinoferrocene (6.0 g; 11 mmoles) in toluene (50 ml) is added a solution of NiCl₂.6H₂O (2.38 g; 10 mmoles) in ethanol (96%; 50 ml) over 10 minutes at room temperature. The suspension is heated to reflux, and zinc dust (2.0 g; 30 mmoles) is added all at once, while vigorously stirring. The mixture is stirred for 1 hr at 60°C and allowed to cool to room temp. After filtration, the air stable residue is suspended in a mixture of 2 M hydrochloric acid in water (50 ml) and ethanol (50 ml). The suspension is stirred at 20°C until the evolution of hydrogen has stopped (about 1.5 hr). The precipitate is filtered, washed successively with 50 ml, 2 x 50 ml ethanol, 50 ml toluene and 2 x 50 ml diethylether, and is then dried in vacuo, to yield 7.20 g (93%) of a crystalline solid. Elem. anal.: C,68.74; H,4.81; Ni,8.2. Calculated for C₄₄H₃₅ClFeNiP₂: C,68.13; H,4.55; Ni,7.57.
Alternatively, the title compound can equally successfully be prepared by reacting diethyl(1,1'-bis-diphenylphosphinoferrocene)-nickel, obtained as described in Example I, with 1-chloro-naphthalene, under the same above reaction conditions.

### Example III

### Preparation of 1,5-cyclooctadiene(1,1'-bis-diphenylphosphinoferrocene)nickel; method A.

The title compound, COD-Ni-dppf, is synthesized under a nitrogen atmosphere; the solvents are deoxygenated before use. To Ni(COD)₂ (0.60 g; 2.2 mmoles) is added benzene (30 ml). The mixture is heated to 40°C until the Ni(COD)₂ is completely dissolved. Then 1,1'-bis-diphenylphosphinoferrocene (1.10 g; 2.0 mmoles) is added in four portions over 5 minutes, while allowing the mixture to cool to room temperature. After stirring at room temperature for 1 hr and at 50°C for 0.5 hr, the mixture is concentrated in vacuo to about 10 ml. Then hexane (70 ml) is added at 60°C, and the mixture is allowed to cool to room temperature and then cooled to -20°C. The precipitate is filtered, washed twice with 50 ml pentane and dried in vacuo to yield 1.25 g (86%) of a crystalline solid. Elem. analysis: C,69.88; H,5.41; Ni,8.5. Calculated for C₄₂H₄₀FeNiP₂: C,69.94; H,5.59; Ni,8.14.

### Example IV

### Preparation of 1,5-cyclooctadiene(1,1'-bis-diphenylphosphinoferrocene)nickel; method B.

In an alternative manner as described in Example III, the title compound is synthesized as follows.
Nitrogen atmosphere; solvents deoxygenated before use. To NiCl₂(dppf) (1.37 g; 2.0 mmoles) are added 1,5-cyclooctadiene (10 ml, 80 mmoles), zinc dust (1.30 g; 20 mmoles) and ethanol (96%; 10 ml). After heating to reflux, the suspension is stirred for 17 hr at room temperature. To the suspension is added methanol (20 ml), and then the suspension is cooled to 5°C. After filtration, the precipitate is washed successively with 2 x 20 ml methanol and 20 ml diethylether. The precipitate is dissolved in 100 ml hot toluene (60°C), filtered and concentrated in vacuo to about 20 ml. Then ethanol (96%; 80 ml) is added, and the suspension is cooled to 5°C. The precipitate is filtered, washed successively with 20 ml ethanol, 20 ml diethylether and 20 ml pentane, and is then dried in vacuo to yield 1.25 g (86%) of a crystalline solid. Elem. analysis: C,69.45; H,5.42; Ni,8.0. Calculated for C₄₂H₄₀FeNiP₂: C,69.94; H,5.59; Ni,8.14.

### Example V

### Cyanation of 4-chlorobenzotrifluoride with 0.2% Ni(dppf)(COD).

The above cyanation is performed under a nitrogen atmosphere. A mixture of Ni(dppf)(COD) (0.72 g; 1.0 mmole), 4-chlorobenzotrifluoride (90 g; 0.50 mole), powdered NaCN (26 g; 0.53 mole), zinc dust (0.65 g; 10 mmoles) and anhydrous THF (75 ml) is heated to 60°C. Then a 0.25 M solution of ZnCl₂ in THF (8 ml; 2 mmoles ZnCl₂) is added all at once, and the mixture is heated to reflux for 24 hrs. After cooling to room temperature the mixture is poured into 500 ml water and successively extracted with 250 ml and 5 x 50 ml diethylether. The combined ethereal solutions are washed twice with 50 ml water, dried over MgSO₄, filtered and concentrated under reduced presure. The residue is distilled through a 30 cm Vigreux column, and yields 77 g (90%) of 4-(trifluoromethyl)benzonitrile as a white solid, b.p. 75°C/15 mm Hg, m.p. 40°C, purity 98% (GLC). The same results are obtained, if instead of the above nickel catalyst diethyl(1,1'-bis-diphenylphosphinoferrocene)nickel in the same molar quantity is used as a catalyst.

### Example VI

### Cyanation of 2-chlorothiophene with 0.5% Ni(dppf)(COD).

The above cyanation is performed under a nitrogen atmosphere. A mixture of Ni(dppf)(COD) (0.72 g; 1.0 mmole), 2-chlorothiophene (23.6 g; 0.20 mole), powdered NaCN (11g; 0.22 mole), zinc dust (1.30 g; 20 mmoles) and anhydrous THF (40 ml) is heated to 60°C. Then a 0.25 M solution of ZnCl₂ in THF (4 ml; 1 mmole ZnCl₂) is added all at once, and the mixture is heated to reflux for 24 hrs to yield 18.5 g 2-thiophenecarbonitrile (85%) (work-up procedure as described in Example V).
The use of diethyl(1,1'-bis-diphenylphosphinoferrocene)nickel in the above cyanation (0.5%) is equally successful.

### Example VII

### Cyanation of 4-chlorobenzotrifluoride with intermediately formed chloro[(4-trifluoromethyl)phenyl](1,1'-bis-diphenylphosphinoferrocene)nickel.

The above cyanation is performed under a nitrogen atmosphere. A mixture of NiCl₂(dppf) (0.34 g; 0.50 mmole), 4-chlorobenzotrifluoride (45 g; 0.25 mole), powdered NaCN (13 g; 0.26 mole) and anhydrous THF (35 ml) is heated to reflux. Then zinc dust (0.33 g; 5.0 mmoles) is added all at once, and the mixture is stirred at reflux for 1 hr. Again zinc dust (0.33 g; 5.0 mmole) is added and the reaction mixture is stirred at reflux for 20 hrs. The conversion, presumably under the influence of the intermediately formed above-defined nickel catalyst, into 4-(trifluoromethyl)benzonitrile, monitored by GLC, is 75%.

### Example VIII

### Cyanation of 4-chlorobenzotrifluoride with 1% Cl-Ni-naphthyl(dppf).

The above cyanation is performed under a nitrogen atmosphere. A mixture of Cl-Ni-naphthyl(dppf) (0.76 g; 1.0 mmole), 4-chlorobenzotrifluoride (18.0 g; 0.10 mole), powdered NaCN (5.60 g; 0.11 mole), zinc dust (0.65 g; 10 mmoles) and anhydrous THF (50 ml) is heated to 60°C. Then a 0.25 M solution of ZnCl₂ in THF (6 ml; 1.5 mmoles ZnCl₂) is added all at once, and the mixture is stirred at reflux for 24 hrs. The conversion into 4-(trifluoromethyl)benzonitrile, monitored by GLC, is 98%.

### Example IX

### Cyanation of 4-chlorohenzotrifluoride with 0.2% Ni(dppf)(COD).

The above cyanation is performed under a nitrogen atmosphere. A mixture of Ni(dppf)(COD) (0.72 g; 1.0 mmole), 4-chlorobenzotrifluoride (90 g; 0.50 mole), powdered NaCN (26 g; 0.53 mole) and anhydrous THF (75 ml) is heated to 60°C. Then a 0.25 M solution of ZnCl₂ in THF (8 ml; 2 mmole ZnCl₂) is added all at once, and the mixture is stirred at reflux (80°C). The rate of conversion into 4-(trifluoromethyl)benzonitrile is satisfactory.

## Claims

1. A nickel compound to be used as a catalyst for cyanation reactions, having the general formula 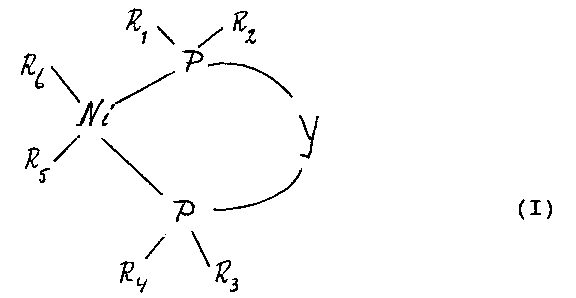 wherein
R₁, R₂, R₃ and R₄ are each independently C₁-C₆ alkyl groups, aryl groups or heteroaryl groups, which (hetero)aryl groups may be substituted with alkyl, halogen, haloalkyl, cyano, amino, alkylamino, N,N-dialkylamino, alkoxy or alkylthio, wherein the alkyl groups have 1 to 4 carbon atoms;
Y is an aliphatic, cycloaliphatic or aromatic bivalent radical having 2 to 18 C atoms;
R₅ is a C₁-C₄ alkyl group, an aryl group or a heteroaryl group, which (hetero)aryl group may be substituted with 1-5 substituents, selected from the group consisting of alkyl, alkoxy, alkylthio, haloalkyl, haloalkoxy, halogen, formyl, alkylcarbonyl, optionally substituted phenyl, optionally substituted phenylcarbonyl, optionally substituted phenoxy, optionally substituted phenoxycarbonyl, optionally substituted phenylalkyl, alkoxycarbonyl and cyano, wherein the alkyl groups have 1 to 8 carbon atoms; and
R₆ is a C₁-C₄ alkyl group, an aryl group or a heteroaryl group, which (hetero)aryl group may be substituted with C₁-C₄ alkyl, or a halogen atom selected from Cl, Br and I;
or wherein R₅ and R₆ together constitute a diene molecule having the general formula
R₉ - CH = CH -(CH₂)ₙ - CH = CH - R₁₀
wherein n is 1-4, and
R₉ and R₁₀ are each C₁-C₈ hydrocarbyl, or
R₉ and R₁₀ together constitute a C₁-C₄ alkylene group;
with the provisos,
(1) that R₆ is alkyl if R₅ is alkyl; and
(2) that R₆ is halogen or (hetero)aryl if R₅ is (hetero)aryl.

2. A nickel compound as claimed in Claim 1, having the general formula 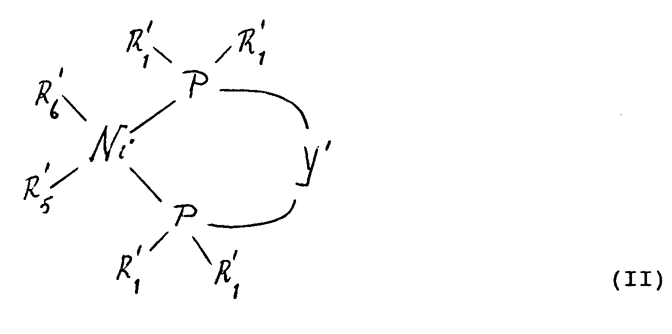 wherein
R₁' is an unsubstituted phenyl group or a phenyl group substituted with C₁-C₄ alkyl, halogen or C₁-C₄ haloalkyl;
Y' is a C₂-C₆ straight or branched alkylene group or a metallocene biradical;
R₅' is a C₁-C₄ alkyl group, an unsubstituted phenyl group or a phenyl group substituted with 1-4 substituents selected from C₁-C₄ alkyl, C₁-C₄ alkoxy, halogen, C₁-C₄ haloalkyl, C₁-C₄ haloalkoxy, formyl, phenyl, phenoxy, C₁-C₄ alkylcarbonyl, cyano, phenylcarbonyl and phenyl(C₁-C₄)alkyl;
or wherein R₅' is a naphthyl group or a heteroaryl group selected from thienyl, pyrrolyl and furyl; and
R₆' is a C₁-C₄ alkyl group or a halogen atom selected from Cl, Br and I; or wherein
R₅' and R₆' together constitute a C₅-C₁₀ cycloalkadiene molecule;
with the provisos,
(1) that R₆' is alkyl if R₅' is alkyl; and
(2) that R₆' is halogen if R₅' is (hetero)aryl.

3. A method of preparing a nickel compound as claimed in Claim 1 or 2,
characterized in that a nickel compound of the general formula 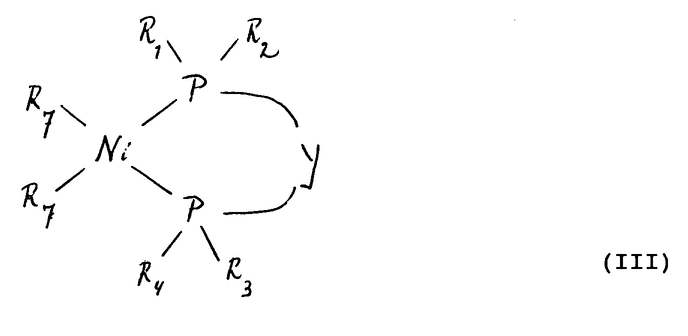 wherein
R₁, R₂, R₃, R₄ and Y have the meanings given in Claim 1, and
R₇ is a C₁-C₄ alkyl group or a (hetero)aryl group,
is prepared by reacting a compound of the general formula 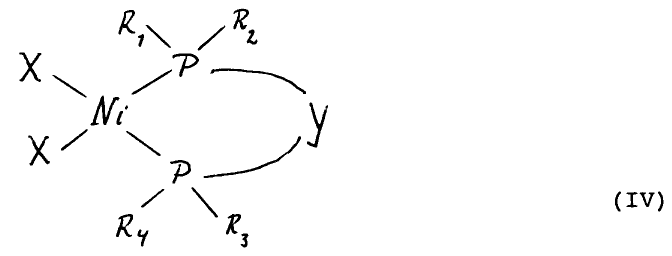 wherein X is a halogen atom selected from Cl, Br and I, with a compound of the general formula
R₇MgX or R₇ZnX (V)

4. A nickel compound which can be used in the method of Claim 3, having the general formula 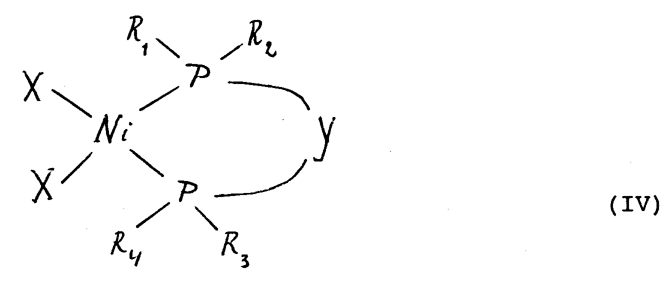 wherein
R₁, R₂, R₃, R₄ and Y have the meanings given in Claim 1, and
X has the meaning given in Claim 3.

5. A method of preparing a nickel compound as claimed in Claim 1 or 2,
characterized in that a nickel compound of the general formula 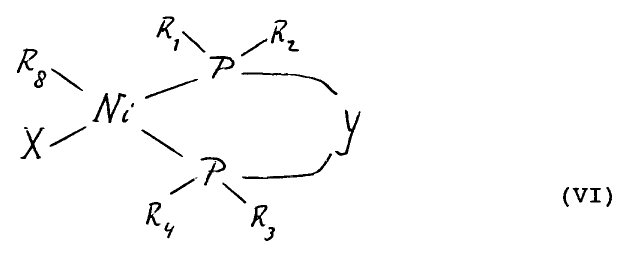 wherein
R₁, R₂, R₃, R₄ and Y have the meanings given in Claim 1,
X has the meaning given in claim 3, and
R₈ is an aryl group or a heteroaryl group, which (hetero)aryl group is optionally substituted with one or more substituents, selected from the group consisting of alkyl, alkoxy, alkylthio, haloalkyl, haloalkoxy, halogen, formyl, alkylcarbonyl, optionally substituted phenyl, optionally substituted phenylcarbonyl, optionally substituted phenoxy, optionally substituted phenoxycarbonyl, optionally substituted phenylalkyl, alkoxycarbonyl and cyano, wherein the alkyl groups have 1 to 8 carbon atoms;
is prepared by reacting a compound of the general formula 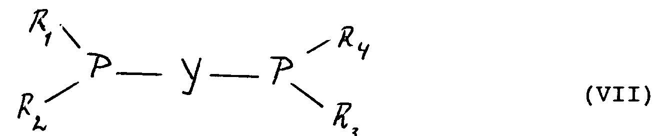 and a compound of the general formula
R₈ - X (VIII)
with NiX₂,
under the influence of a reducing agent.

6. A method of preparing a nickel compound as claimed in Claim 1 or 2,
characterized in that a nickel compound of the general formula 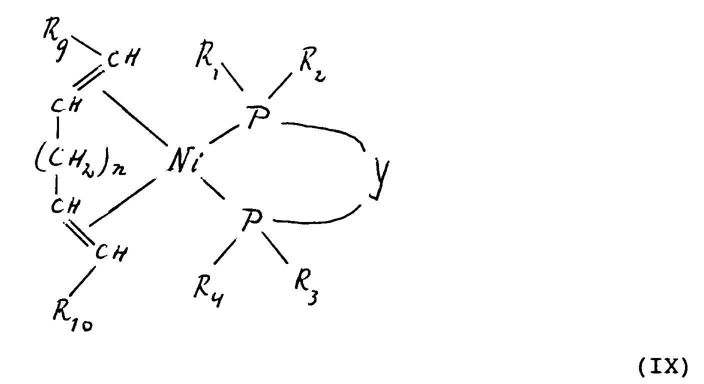 wherein the symbols have the meanings given in Claim 1,
is prepared, either
(i) by reacting a nickel complex of the general formula 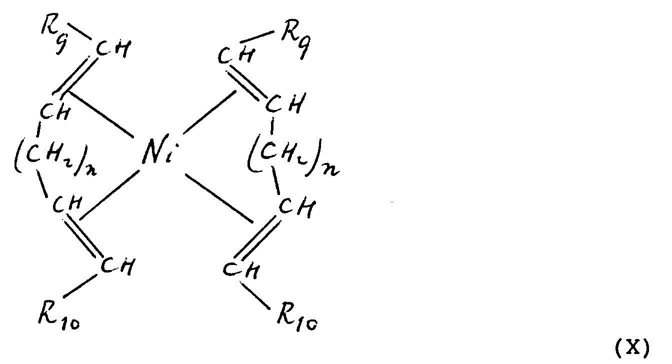 with a compound of the general formula VII, as presented in Claim 6; or
(ii) by reacting a compound of the general formula IV, as presented in Claim 3, wherein X has the meaning given in Claim 3, with a compound of the general formula
R₉ - CH = CH -(CH₂)ₙ - CH = CH -R₁₀ (XI)
under the influence of a reducing agent.

7. Use of a nickel compound as claimed in Claim 1 or 2 for the conversion of an aromatic halide of the general formula
Ar - X (XII)
wherein
Ar is an aryl or heteroaryl group, which groups may be substituted with 1-5 substituents, selected from the group consisting of alkyl, alkoxy, alkylthio, haloalkyl, haloalkoxy, halogen, formyl, alkylcarbonyl, optionally substituted phenyl, optionally substituted phenylcarbonyl, optionally substituted phenoxy, optionally substituted phenoxycarbonyl, optionally substituted phenylalkyl, alkoxycarbonyl and cyano, wherein the alkyl groups have 1 to 8 carbon atoms; and
X has the meaning given in Claim 3;
into an aromatic cyanide of the general formula
Ar - CN (XIII)
under the influence of an alkali metal cyanide.

8. A method of preparing an aromatic cyanide of the general formula
Ar - CN (XIII)
wherein Ar has the meaning given in Claim 7,
characterized in that an aromatic halide of the general formula
Ar - X (XII)
wherein X has the meaning given in Claim 3,
is reacted with an alkali metal cyanide in a substantially anhydrous non-dipolar aprotic solvent under the influence of a phosphine-nickel catalyst and in the presence of a salt of a transition metal.

9. A method as claimed in Claim 8, characterized in that the solvent is selected from tetrahydrofuran, dioxan and methyl tert.-butyl ether, and in that the metal salt is zinc chloride.

10. A method as claimed in Claim 8 or 9, characterized in that the reaction is performed in the presence of powdered zinc.
